# EUROPÄISCHE PATENTANMELDUNG

(11) **EP 4 700 006 A1**
(43) Veröffentlichungstag der Anmeldung: **25.02.2026**
(21) Anmeldenummer: 24195345.4
(22) Anmeldetag: 20.08.2024
(51) Int. Cl.: C07C 45/50, C07F 9/6574

(54) **BISPHOSPHITE MIT EINEM ETHER-REST AM FLÜGELBAUSTEIN**

(71) Anmelder: Evonik Oxeno GmbH & Co. KG, 45772 Marl (DE)
(72) Erfinder: BÜKER,Julia, 44787 Bochum (DE); FRANKE, Robert, 45772 Marl (DE); FRIDAG, Dirk, 45721 Haltern am See (DE); BILKE, Marius, 45711 Datteln (DE); BRUNS, Bastian, 44791 Bochum (DE); BÜRK, Vincent, 45665 Recklinghausen (DE); KUCMIERCZYK, Peter, 44628 Herne (DE); MARKOVIC, Ana, 45721 Haltern am See (DE); PESCHKE, Roland, 47057 Duisburg (DE); WESSNER, Maximilian, 44139 Dortmund (DE); SALE, Anna Chiara, 40474 Düsseldorf (DE)
(74) Vertreter: Evonik Patent Association

(57) **Zusammenfassung**

Bisphosphite mit einem Ether-Rest am Flügelbaustein.

## Beschreibung

Die Erfindung betrifft Bisphosphite mit einem Ether-Rest am Flügelbaustein. Des Weiteren betrifft die Erfindung die Verwendung der Bisphosphite in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

In WO 02/00670 A1 werden Bisphosphitverbindungen, deren Matallkomplexe und Verwendung der Verbindungen und Komplexe in der Olefinhydroformylierung beschrieben. Unter anderem wird hier die Verbindung (Ila) gezeigt:

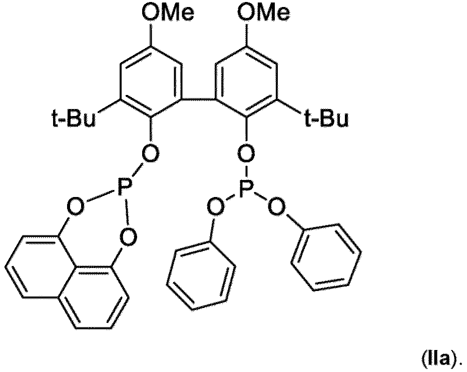

Die technische Aufgabe der Erfindung ist die Bereitstellung einer Verbindung, mit welcher bei der Hydroformylierung von Olefinen eine gute Ausbeute erzielt werden kann.

Die Aufgabe wird gelöst durch eine Verbindung gemäß Anspruch 1.

Verbindung gemäß Formel (I): 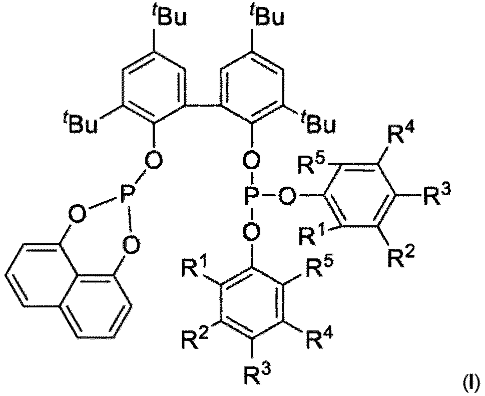
wobei R¹, R², R³, R⁴, R⁵ ausgewählt sind aus: -H, -X,
wobei X für den folgenden Rest steht: 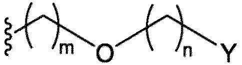
m und n für eine Zahl von 0 bis 5 stehen,
und Y ausgewählt ist aus: -CH₃, -Ph,
und mindestens einer der Reste R¹, R², R³, R⁴, R⁵ nicht für -H steht.

In einer Ausführungsform stehen R¹ und R⁵ für -H.

In einer Ausführungsform stehen mindestens drei der Reste R¹, R², R³, R⁴, R⁵ für -H.

In einer Ausführungsform stehen vier der Reste R¹, R², R³, R⁴, R⁵ für -H.

In einer Ausführungsform stehen R¹, R², R⁴, R⁵ für -H.

In einer Ausführungsform stehen m und n für eine Zahl von 0 bis 3.

In einer Ausführungsform steht entweder m oder n für 0.

In einer Ausführungsform steht Y für -Ph.

In einer Ausführungsform steht Y für -CH₃.

In einer Ausführungsform weist die Verbindung eine der Strukturen (1) oder (2) auf:

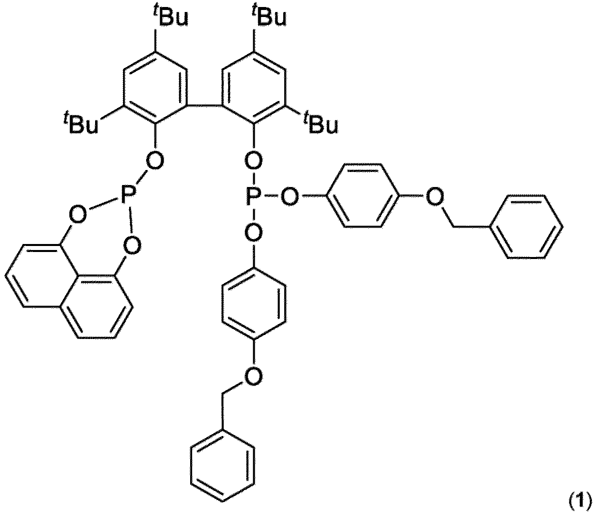

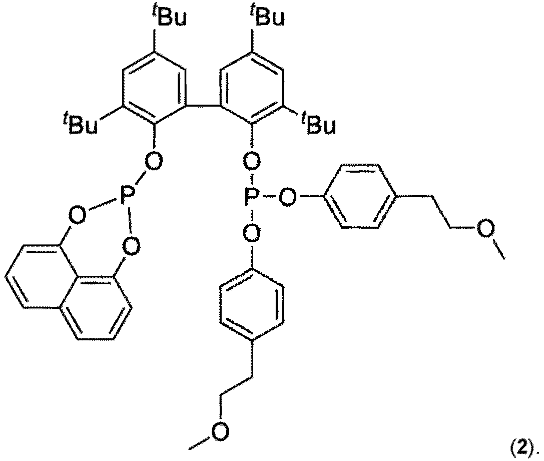

In einer Ausführungsform weist die Verbindung die Struktur (1) auf:

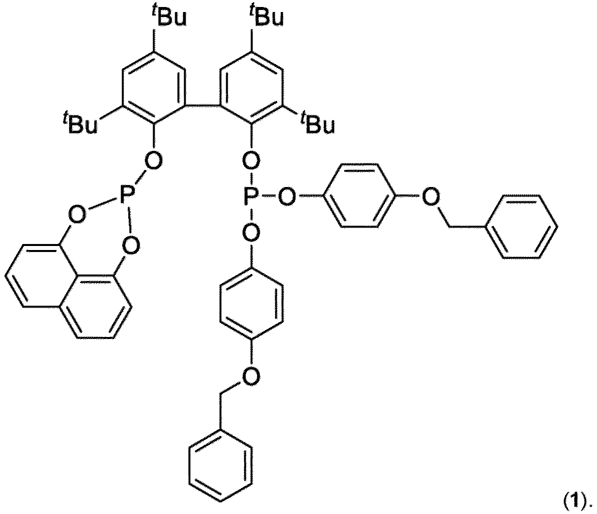

In einer Ausführungsform weist die Verbindung die Struktur (2) auf:

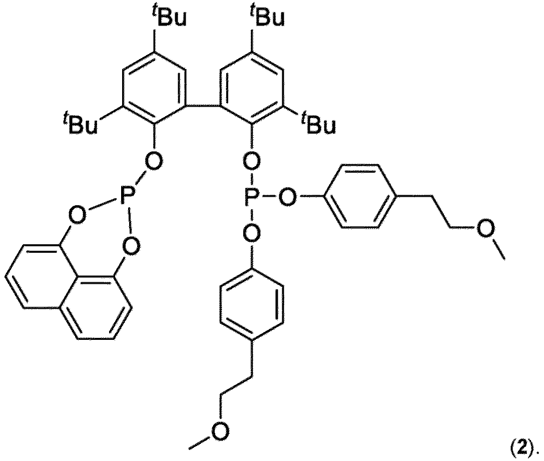

Neben den Verbindungen selbst wird auch ein Verfahren beansprucht, in dem die zuvor beschriebenen Verbindungen zum Einsatz kommen.

Verfahren umfassend die Verfahrensschritte:
a) Vorlegen eines Olefins;
b) Zugabe der zuvor beschriebenen Verbindung;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

In einer Variante des Verfahrens ist die Substanz, welche Rh umfasst, ausgewählt aus: Rh(acac)(CO)₂, Rh(acac)(cod) (Umicore, acac = Acetylacetonat-Anion; cod = 1 ,5-Cyclooctadien), Rh₄CO₁₂.

In einer Variante des Verfahrens ist die Substanz, welche Rh umfasst, Rh(acac)(CO)₂.

Im Folgenden soll die Erfindung anhand von Ausführungsbeispielen näher erläutert werden.

### Synthese

### Erste Stufe

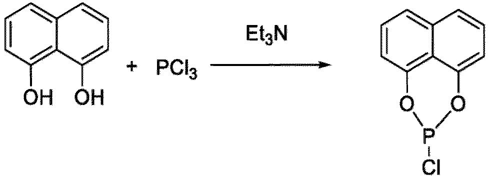

Es wurden 0,076 mol Naphthalin-1,8-diol über Nacht bei 50 °C mittels Ölpumpenvakuum getrocknet. Am darauf folgenden Tag wurde der Schlenk mit Argon geflutet und das Naphthalin-1,8-diol in 350 ml getrocknetem Toluol gelöst. In einem sekurierten Schlenk wurden 0,114 mol Phosphortrichlorid in 120 ml getrocknetem Toluol gelöst. Anschließend wurde die Naphthalin-1,8-diol-Lösung bei -20 °C langsam und stetig zur PCI₃-Lösung getropft. Danach wurden 0,165 mol Triethylamin langsam unter hoher Rührgeschwindigkeit bei -20 °C zu der Lösung hinzugetropft. Die Lösung wurde auf Raumtemperatur gebracht und über Nacht weiter gerührt. Am nächsten Tag wurde die Reaktionsmischung abgefrittet, der Filterkuchen mit zweimal je 25 ml Toluol gewaschen und das Filtrat mittels Ölpumpenvakuum bei 40 °C eingeengt.

### Ausbeute: 86%

### Zweite Stufe

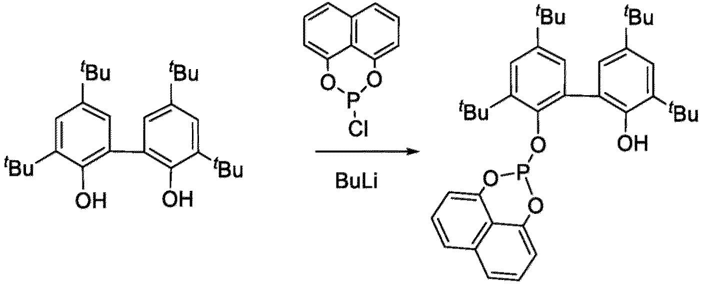

Es wurden 0,016 mol Biphenol eingewogen, über Nacht mittels Ölpumpenvakuum getrocknet und am nächsten Morgen mit Argon geflutet. Das Biphenol wurde in 40 mL Toluol gelöst. Unter Inertgasatmosphäre wurden 0,016 mol Chlorophosphit eingewogen, in 40 mL Toluol gelöst und mit 0,016 mol entgastem Triethylamin versetzt. Die Chlorophosphit-Toluol-Lösung wurde bei Raumtemperatur über 1 h zu der Biphenol-Lösung hinzugetropft und bei 40 °C für 24 h gerührt. Die Reaktionsmischung wurde abgefrittet und der Filterkuchen zweimal mit je 20 ml Toluol nachgewaschen. Das erhaltene Filtrat wurde unter Ölpumpenvakuum bei 40 °C eingeengt und getrocknet.

| | |
|---|---|
| Ausbeute: | 75% |

### Dritte Stufe

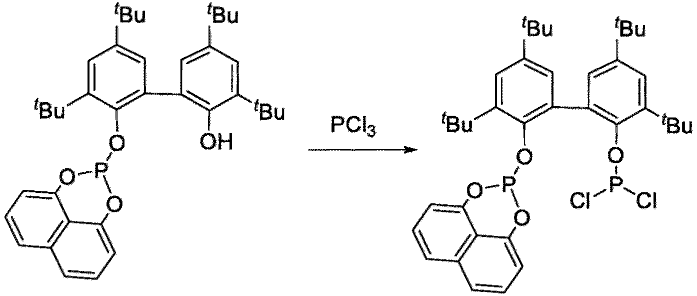

Unter Inertgasatmosphäre wurden 11,9 mmol des Monophosphits abgewogen und in 150 ml getrocknetem Toluol und 29,8 mmol entgastem Triethylamin gelöst. In einem sekurierten Schlenk wurden 14,9 mmol Phosphortrichlorid in 100 ml getrocknetem Toluol gelöst und auf 0 °C abgekühlt. Im Anschluss wurde die Organochlorophosphit-Triethylamin-Lösung bei 0 °C zu der Phosphortrichlorid-Lösung gegeben. Die Reaktionsmischung wurde bei Raumtemperatur für 24 h gerührt. Das entstandene Ammoniumhydrochlorid wurde abgefrittet und zweimal mit je 50 ml getrocknetem Toluol gewaschen. Das erhaltene Filtrat wurde anschließend unter Ölpumpenvakuum bei 45 °C bis zur Trockene eingeengt.

| | |
|---|---|
| Ausbeute: | 87% |

### Synthese (2)

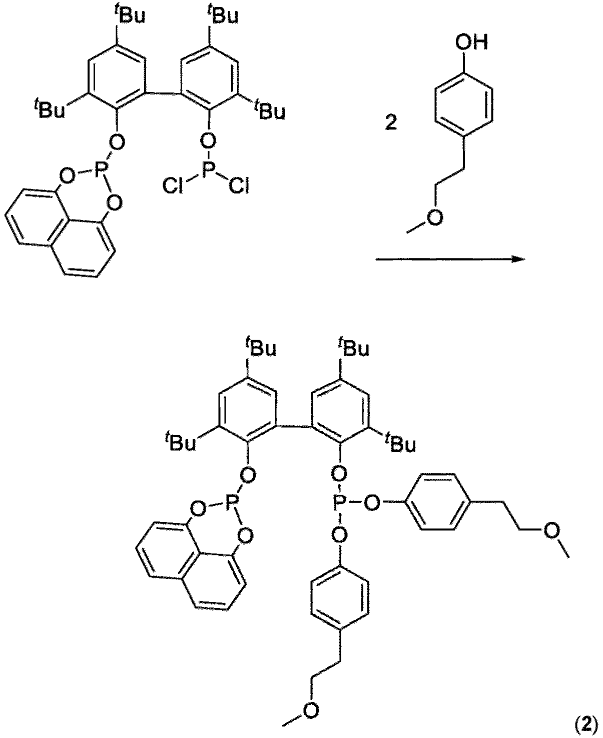

Unter Inertgasatmosphäre wurden 2,9 mmol Organodichlorophosphit abgewogen und in 30 ml getrocknetem Toluol suspendiert. Es wurden 6,7 mmol des Phenol in einem Schlenk abgewogen und kurz mittels Ölpumpenvakuum sekuriert. Anschließend wurde der Schlenk mit Argon geflutet und das Phenol in 20 ml getrocknetem Toluol gelöst und 14,3 mmol entgastes Triethylamin hinzugegeben. Anschließend wurde die Phenol-Lösung langsam und stetig bei Raumtemperatur zur Chlorophosphit-Suspension gegeben. Die Reaktionslösung wurde über Nacht bei Raumtemperatur gerührt. Das entstandene Ammoniumhydrochlorid wurde abgefrittet und zweimal mit je 10 ml getrocknetem Toluol nachgewaschen. Das erhaltene Filtrat wurde anschließend mittels Ölpumpenvakuum bei 40 °C bis zur Trockene eingeengt. Das getrocknete Filtrat wurde mittels Säulenchromatographie aufgereinigt.
Ausbeute: 45 %

Die Verbindung **(1)** wurde analog hergestellt.

Ebenso die Vergleichsverbindung **(IIa).**

### Katalyseversuche

Es wird unter Argon-Atmosphäre gearbeitet. Reaktionsgefäße sind zuvor unter Einwirkung von Temperatur (80 °C) und Ölpumpenvakuum getrocknet worden. Flüssige Substanzen wurden für mind. 15 Minuten durch Einperlen von Argon entgast. Die Hydroformylierung wurde in einem mit Druckkonstanthaltung ausgestatteten 0,5 L-Autoklaven der Firma Berghof Products + Instruments GmbH, durchgeführt. Beheizt wird der Reaktor mittels Ölbad der Firma IKA. Der Reaktor dient zum Gasaustausch und als Temperierwerk. Innerhalb dieses Reaktors wurden fünf Glasvials (20mL), gefüllt mit Katalysatorlösung und Magnetrührfischen unter Argon gebördelt, so platziert, dass ein Gasaustausch zwischen Vials und Reaktorraum möglich ist. Durch im Reaktor enthaltenes Thermalöl wurden die Glasvials temperiert. Angegebene Reaktionstemperaturen wurden im inneren der Glasvials gemessen. Als Substrat eingesetzt wurde n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %).

Für einen Versuchslauf wurde eine Stammlösung vorab unter Argonatmosphäre vorbereitet. Hierfür wurden 0,0127 g Rh(acac)(CO)₂ und die entsprechende Menge an Phosphitverbindung (MV Lig:Rh = 5:1) eingewogen und mit 48,0 ml Toluol aufgefüllt. Von dieser Lösung wurden je ca. 8 mL auf die Vials verteilt und die genaue Menge gewogen. Die Vials wurden im Reaktor platziert und dieser verschlossen. Es wurde dreimal mit Argon und dreimal mit Synthesegas (Linde; H₂ (99,999 %) : CO (99,997%) = 1:1) gespült. Nach erfolgter Druckprüfung wurde der Autoklav bei einem Gesamtdruck von 10 bar unter Rühren (900 U/min) auf die angestrebte Temperatur von 120°C aufgeheizt. Nach Erreichen der Reaktionstemperatur wurde der Synthesegasdruck auf 20 bar erhöht und das Substrat mittels HPLC-Pumpe mit je 2 mL zum Reaktionsstart zudosiert. Daraus ergibt sich eine Rh-Konzentration von 100 ppm. Nach 1 h Reaktionsstart bei konstantem Druck wurde eine Probe je Vial gezogen und unverdünnt gaschromatographisch analysiert: HP 6890, Petrocol^{®} DH 150, 150 m x 0,25 mm x 1 µm. Die quantitative Bestimmung von Restolefin und Aldehyd erfolgte gegen das Lösungsmittel Toluol als interner Standard. Die in der untenstehenden Tabelle gelisteten Ergebnisse stellen den Mittelwert über einen Versuchslauf da.

### Ergebnisse der Katalyseversuche

[Rh]: 100 ppm, p: 20 bar, T: 120 °C; t: 1 h

Das eingesetzte n-Octen-Gemisch bestand aus den C8-Isomeren: 1-Octen, cis-2-Octen, trans-2-Octen, cis-3-Octen, trans-3-Octen, cis-4-Octen und trans-4-Octen.

**Tabelle 1:**

| Ligand | Ausbeute [%] |
|---|---|
| **(1)** | 44 |
| **(2)** | 40 |
| **(IIa)*** | 39 |

| | |
|---|---|
| * nicht erfindungsgemäßes Ausführungsbeispiel | |

Die durchgeführten Versuche belegen, dass die gestellte Aufgabe durch die erfindungsgemäßen Verbindungen gelöst wird.

## Patentansprüche

1. Verbindung gemäß Formel (I): 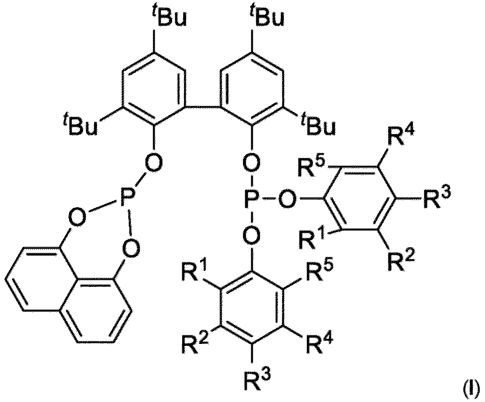
wobei R¹, R², R³, R⁴, R⁵ ausgewählt sind aus: -H, -X,
wobei X für den folgenden Rest steht: 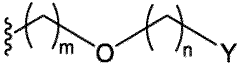
m und n für eine Zahl von 0 bis 5 stehen,
und Y ausgewählt ist aus: -CH₃, -Ph,
und mindestens einer der Reste R¹, R², R³, R⁴, R⁵ nicht für -H steht.

2. Verbindung nach Anspruch 1,
wobei R¹ und R⁵ für -H stehen.

3. Verbindung nach einem der Ansprüche 1 oder 2,
wobei mindestens drei der Reste R¹, R², R³, R⁴, R⁵ für -H stehen.

4. Verbindung nach einem der Ansprüche 1 bis 3,
wobei vier der Reste R¹, R², R³, R⁴, R⁵ für -H stehen.

5. Verbindung nach einem der Ansprüche 1 bis 4,
wobei R¹, R², R⁴, R⁵ für -H stehen.

6. Verbindung nach einem der Ansprüche 1 bis 5,
wobei m und n für eine Zahl von 0 bis 3 stehen.

7. Verbindung nach einem der Ansprüche 1 bis 6,
wobei Y für -Ph steht.

8. Verbindung nach einem der Ansprüche 1 bis 6,
wobei Y für -CH₃ steht.

9. Verbindung nach einem der Ansprüche 1 bis 6,
wobei die Verbindung eine der Strukturen **(1)** oder **(2)** aufweist:

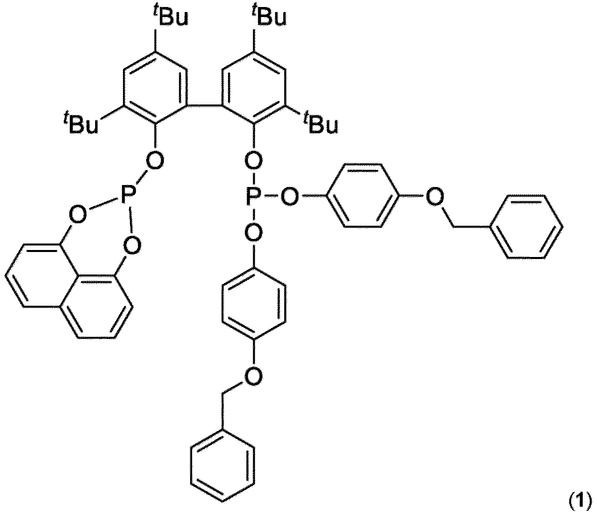

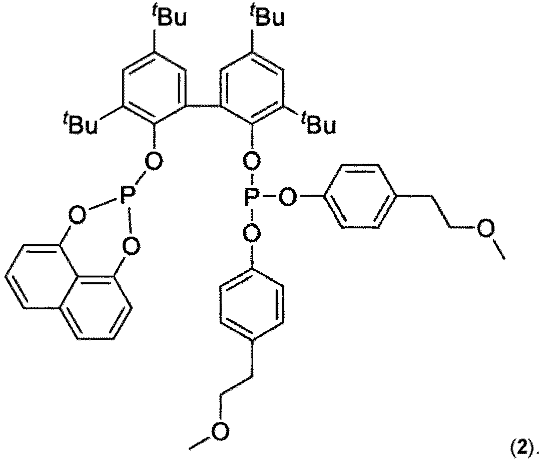

10. Verbindung nach einem der Ansprüche 1 bis 6,
wobei die Verbindung die Struktur (1) aufweist:

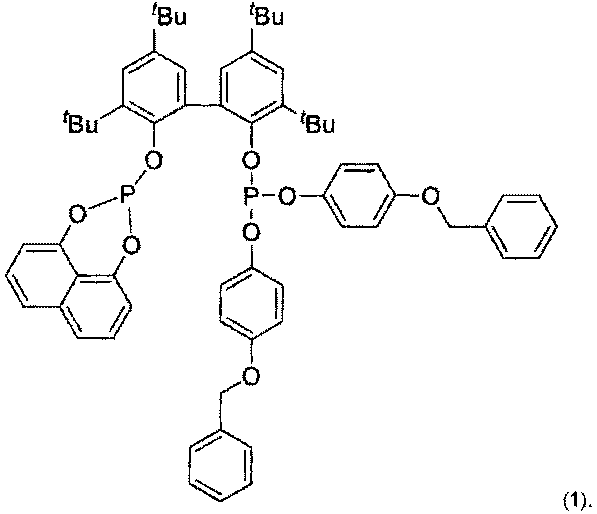

11. Verbindung nach einem der Ansprüche 1 bis 6,
wobei die Verbindung die Struktur **(2)** aufweist:

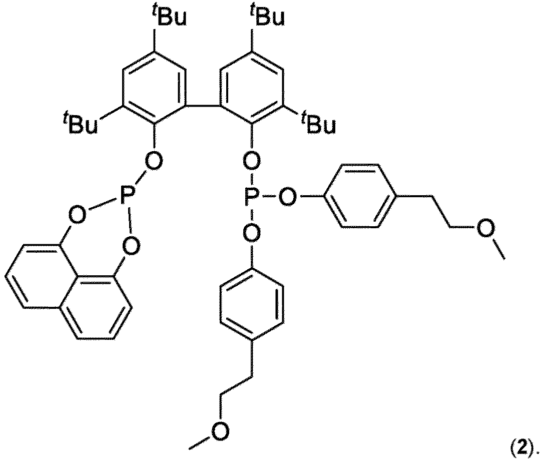

12. Verfahren umfassend die Verfahrensschritte:
a) Vorlegen eines Olefins;
b) Zugabe einer Verbindung nach einem der Ansprüche 1 bis 11;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

13. Verfahren nach Anspruch 12,
wobei die Substanz, welche Rh umfasst, ausgewählt ist aus: Rh(acac)(CO)₂, Rh(acac)(cod) (Umicore, acac = Acetylacetonat-Anion; cod = 1,5-Cyclooctadien), Rh₄CO₁₂.

14. Verfahren nach einem der Ansprüche 12 oder 13,
wobei die Substanz, welche Rh umfasst, Rh(acac)(CO)₂ ist.
